# EUROPEAN PATENT SPECIFICATION

(11) **EP 3 642 631 B1**
(45) Date of publication and mention of the grant of the patent: **30.03.2022**
(21) Application number: 18730396.1
(22) Date of filing: 19.06.2018
(51) Int. Cl.: G01N 33/86

(54) **METHODS FOR IDENTIFYING WHETHER PATIENTS WITH ACUTE DECOMPENSATED HEART FAILURE (ADHF) EXHIBIT A HYPERCOAGULABLE STATE**
VERFAHREN ZUR ERKENNUNG, OB PATIENTEN MIT AKUTER DEKOMPENSIERTER HERZINSUFFIZIENZ (ADHF) EINEN HYPERKOAGULIERBAREN ZUSTAND AUFWEISEN
PROCÉDÉS PERMETTANT DE DIAGNOSTIQUER LES PATIENTS SOUFFRANT D'INSUFFISANCE CARDIAQUE DÉCOMPENSÉE AIGUË (ADHF) PRÉSENTENT UN ÉTAT D'HYPERCOAGULABILITÉ

(30) Priority: 20.06.2017 EP 17305753
(43) Date of publication of application: 29.04.2020
(73) Proprietor: INSERM (Institut National de la Santé et de la Recherche Médicale), 75013 Paris (FR); Université de Lorraine, 54000 Nancy (FR); Centre Hospitalier Et Universitaire De Nancy (CHU), 54000 Nancy (FR)
(72) Inventor: REGNAULT, Véronique, 54505 Vandoeuvre les Nancy cedex (FR); POPOVIC, Batric, 54500 Vandoeuvre les Nancy (FR); LACOLLEY, Patrick, 54505 Vandoeuvre les Nancy cedex (FR)
(74) Representative: Inserm Transfert
(86) International application number: PCT/EP2018/066261
(87) International publication number: WO 2018/234308

(56) References cited:
- TOURNIER A ET AL: "Calibrated automated thrombography demonstrates hypercoagulability in patients with idiopathic pulmonary arterial hypertension", THROMBOSIS RESEARCH, TARRYTOWN, NY, US, vol. 126, no. 6, 1 December 2010 (2010-12-01), pages e418-e422, XP027534352, ISSN: 0049-3848 [retrieved on 2010-11-29]
- ZANNAD FAIEZ ET AL: "Is thrombosis a contributor to heart failure pathophysiology? Possible mechanisms, therapeutic opportunities, and clinical investigation challenges", INTERNATIONAL JOURNAL OF CARDIOLOGY, vol. 167, no. 5, 2013, pages 1772-1782, XP028703424, ISSN: 0167-5273, DOI: 10.1016/J.IJCARD.2012.12.018
- MINAMI YUICHIRO ET AL: "Elevated D-dimer levels predict an adverse outcome in hospitalized patients with acute decompensated heart failure", INTERNATIONAL JOURNAL OF CARDIOLOGY, vol. 204, 2015, pages 42-44, XP029366945, ISSN: 0167-5273, DOI: 10.1016/J.IJCARD.2015.11.156
- KIM JU H ET AL: "Coagulation Abnormalities in Heart Failure: Pathophysiology and Therapeutic Implications", CURRENT HEART FAILURE REPORTS, CURRENT SCIENCE INC., PHILADELPHIA, PA, US, vol. 13, no. 6, 4 November 2016 (2016-11-04), pages 319-328, XP036113994, ISSN: 1546-9530, DOI: 10.1007/S11897-016-0308-6 [retrieved on 2016-11-04]
- NARANG AKHIL ET AL: "Embolic Stroke in Cardiomyopathy Should Patients be Anticoagulated?", CARDIOLOGY CLINICS, W.B. SAUNDERS COMPANY, PHILADELPHIA, US, vol. 34, no. 2, 1 May 2016 (2016-05-01), pages 215-224, XP009195230, ISSN: 0733-8651
- TRIPODI ARMANDO ET AL: "Hypercoagulability in patients with Cushing disease detected by thrombin generation assay is associated with increased levels of neutrophil extracellular trap-related factors", ENDOCRINE, HUMANA PRESS, INC, US, vol. 56, no. 2, 22 July 2016 (2016-07-22), pages 298-307, XP036214261, ISSN: 1355-008X, DOI: 10.1007/S12020-016-1027-1 [retrieved on 2016-07-22]

## Description

### FIELD OF THE INVENTION:

The present invention relates to methods for identifying whether patients with acute decompensated heart failure (ADHF) exhibit a hypercoagulable state.

### BACKGROUND OF THE INVENTION:

Despite the benefits observed with recent therapies including neurohormonal modulators, the prognosis of patients hospitalized for acute decompensated heart failure (ADHF) remains poor, with readmission rates approximating 50% at 6 months¹ and 1-year mortality up to 30%.² This very negative prognosis is partly influenced by thromboembolic events which occur in up to 30% of patients and are often the cause of death.³ These epidemiological findings are supported by multiple mechanisms that may contribute to a hypercoagulable state in patients with heart failure (HF) such as enhancement of soluble procoagulant factors, endothelial dysfunction, or inflammation.^{4,5} Therefore, thrombotic mechanisms may emerge as potential therapeutic targets of interest in HF.^{5,6} Targeted reduction of thrombin generation with a new oral anticoagulant, rather than non-selective depletion of multiple vitamin K-dependent clotting factors with warfarin, may hold promise. Such a randomized trial, COMMANDER-HF including approximatively 5000 patients is under-way and initiates anticoagulant therapy with the anti-Xa rivaroxaban agent shortly after discharge from ADHF.⁶ To support such studies, a biological assessment of excessive thrombin generation in ADHF patients and a better comprehension of the mechanisms leading to thrombin formation in longitudinal studies including selected patients and using sophisticated tests are required.

There is increasing evidence that endothelial damage contributes to the pathogenesis of ADHF. The endothelium exerts its function in maintaining vascular homeostasis through the balance between its natural anticoagulant function supported by the activated protein C (APC) pathway and induced procoagulant activities. At the endothelial surface, binding of thrombin to thrombomodulin (TM) results in the activation of protein C and this reaction is facilitated when protein C is bound to the endothelial protein C receptor (EPCR). It has been reported that disruption of this endothelial-dependent APC pathway promotes endocardial thrombosis in ADHF in mice.⁷ High levels of endothelial-derived extracellular vesicles (eEVs) reflecting advanced endothelial function are independent predictors of cardiovascular events in HF patients.⁸ This is in favor also of procoagulant properties of endothelium in HF. Finally, neutrophil extracellular traps, which are released in a variety of diseases, induce endothelial cell damage and proteolytically degrade the endothelium-dependent anticoagulant system such as TFPI, protein C and TM.⁹

### SUMMARY OF THE INVENTION:

The present invention relates to methods for identifying whether patients with acute decompensated heart failure (ADHF) exhibit a hypercoagulable state. The present invention is defined by the claims.

### DETAILED DESCRIPTION OF THE INVENTION:

Subjects with heart failure (HF) are at higher risk of developing thrombosis. The inventors thus determined the thrombin generating capacity in patients with acute decompensated heart failure (ADHF). Their prospective study included 34 ADHF patients and 30 control inpatients without HF. In vitro thrombin generation and its downregulation by activated protein C (APC) was monitored by a calibrated automated thrombogram at hospital admission, on the day of discharge and after at least six weeks of clinical stability. Circulating endothelium-derived extracellular vesicles (eEVs) were quantified by flow cytometry. Circulating levels of DNA-histone complexes were quantified by ELISA. Compared to controls, endogenous thrombin potential (ETP) was higher in ADHF patients at admission using platelet-rich plasma and remained increased during the hospitalization period. After six weeks of clinical stability, ETP values were lowered to values of controls. The APC concentration inhibiting thrombin generation by 50% was higher at admission compared with the post-discharge period. Soluble markers of endothelial dysfunction including von Willebrand factor, factor VIII and endothelial protein C receptor were higher in ADHF patients at admission. The plasma levels of DNA-histone complexes were elevated significantly in ADHF patients at admission compared to controls and decreased significantly at the post-discharge point. Higher concentrations (5-fold increase) of annexin-V/tissue factor (TF)-positive eEVs were found also in ADHF patients at admission compared to controls and decreased significantly at the post-discharge point. The concentration of TF-positive eEVs was correlated positively with ETP. Advanced endothelial dysfunction resulting in raised procoagulant eEVs and FVIII levels increases thrombin generation and dampens down-regulation by the APC pathway at the acute phase of decompensated HF. The findings demonstrate that methods of determining the thrombin generating capacity in patients with acute decompensated heart failure (ADHF), which combine measurements of endogenous thrombin potential and endothelial dysfunction, are particularly valuable for stratifying the patients and thus for orienting treatments and following-up of the patients.

Accordingly, the first object of the present invention relates to a method for identifying whether a patient with acute decompensated heart failure (ADHF) exhibits a hypercoagulable state comprising i) determining thrombin generation capacity from a blood sample obtained from the patient, ii) determining the level of at least one soluble biomarker of endothelial dysfunction, iii) comparing the capacity determined at step i) and the level determined at step ii) with their respective predetermined reference values and iv) concluding that the patient exhibits a hypercoagulable state when both the thrombin generation capacity and the level of soluble marker of endothelial dysfunction are higher than their respective predetermined reference values.

As used herein, the term "heart failure" denotes the inability of the heart to supply sufficient blood flow to meet the body's needs and this pathology is well-described in medical practice. As used herein, the term "acute heart failure" denotes sudden onset heart failure, referring to episodes in which a patient with known chronic heart failure or devoid of chronic heart failure abruptly develops worsening symptoms and requires hospitalization. Common symptoms of complications due to acute heart failure include, but are not limited to, dyspnea due to pulmonary congestion or cardiogenic shock due to low cardiac output, easy fatigueability (exercise intolerance), peripheral edema, anasarca (pronounced generalized edema), nocturia (frequent nighttime urination), bradycardia, heart block, hypotension, dizziness, syncope, diabetes, oliguria or anuria, hypokalemia, bronchospasm, cold sweat, and asthma. As used herein, the term "acute decompensated heart failure" or "ADHF" has its general meaning in the art and refers to a state whereby a patient suffering from heart failure experiences increased signs and symptoms of the disease after a period of relative stability (Allen and O'Connor, CMAJ 176(6):797-805, 2007).

As used herein, the term "hypercoagulable state" has its general meaning in the art and refers to the medical term for a condition in which there is an abnormally increased tendency toward blood clotting (coagulation) compared to age-matched control subjects. In particular, the hypercoagulable state indicates that the patient is at high risk of thrombosis. As used herein, the term "thrombosis" refers to the formation of a blood clot inside a blood vessel, obstructing the flow of blood through the circulatory system. Thus the term "thrombosis" includes inter alia atrophic thrombosis, arterial thrombosis, cardiac thrombosis, coronary thrombosis, creeping thrombosis, mesenteric thrombosis, placental thrombosis, propagating thrombosis, traumatic thrombosis and venous thrombosis.

The term "blood sample" means a whole blood sample obtained from the patient. For example, blood may be drawn from the patient following a standard venipuncture procedure in vacutainers containing 0.10⁶ M sodium citrate. In some embodiments, the bood sample is a platelet-rich plasma (PRP) sample or a platelet-poor plasma sample. For instance, platelet-rich plasma (PRP) may then be obtained by centrifugation at 190 g for 10 min (room temperature) followed by pipetting of the PRP layer.

According to the present invention, the thrombin generation capacity is determined by any method well known in the art. For instance, using a calibrated automated thrombogram (CAT) assay e.g. developed by Hemker and co-workers, thrombin generation in plasma is accurately measured (Hemker HC, Giesen P, Al Dieri R, Regnault V, de Smedt E, Wagenvoord R, Lecompte T, Béguin S. Calibrated automated thrombin generation measurement in clotting plasma. Pathophysiol Haemost Thromb. 2003;33(1):4-15). Calibrated automated thrombogram displays the concentration of thrombin in clotting plasma with or without platelets (platelet-rich plasma/platelet-poor plasma, PRP/PPP) by monitoring the splitting of a fluorogenic substrate and comparing it to a constant known thrombin activity in a parallel, non-clotting sample. Typically, thrombin activity against time is thus presented as a curve of Thrombogram^{™} (TG) which is characterized by an initiation phase providing minute amounts of thrombin followed by a burst of activity. Blood forms a clot at the very beginning of the burst and almost all thrombin is formed after the clot has formed. The Thrombogram^{™} thus shows various phases including lag time, propagation phase, time to peak and peak height and ETP. The term "lag time" as used herein is meant to indicate the time (min) it takes before the thrombin burst starts. The term "time to peak" (min), as used herein, means the time between the start of the reaction and the moment that the peak is reached. As used herein, the term "peak height" (nM) means the maximal thrombin activity attained during the assay on a tested sample. The term "ETP" or "Endogenous Thrombin Potential", as used herein, means the time integral of the curve until the moment of (near)zero thrombin activity is attained, corresponding to the area under the curve (nM. min) (see e.g. EP 0 420 332-B1, EP 0 802 986-B1 and Hemker et al. (1993) Thromb. Haemostasis 70: 617-624) ETP thus represents the balance between pro- and anti-coagulant forces operating in plasma and is preferably calculated for determining the patient's thrombin generation capacity. Previously known evaluation methods for determining the endogenous thrombin potential include those disclosed in Hemker et al. (1993) Thromb. Haemost 70: 617-624; and in WO 2004/016807-A1 ad typically include the one described in EXAMPLE. Accordingly, in some embodiments, the method of the present invention comprises determining the ETP in the blood sample obtained from the patient and comparing said value with a predetermined reference value. In some embodiments, activated protein C (APC) is added to the blood sample and the APC concentration inhibiting thrombin generation by 50% is thus determined and compared to a predetermined reference value as described in the EXAMPLE.

The method of the present invention comprises determining the level of at least one soluble marker of endothelial dysfunction selected from the group consisting of von Willebrand factor, factor VIII, endothelial protein C receptor, tissue factor pathway inhibitor, endothelium-derived extracellular vesicles and circulating DNA-histone complexes in the blood sample from the patient.

As used herein, the term "Von Willebrand factor" has its general meaning in the art and refers to any soluble polypeptide present in the blood sample that is derived from the human Von Willebrand factor, which is characterized by the NCBI reference sequence NP_000543.

As used, the term "factor VIII" or "FVIII" has its general meaning in the art and refers to any soluble polypeptide present in the blood sample that is derived from human Factor VIII, which is characterized by the NCBI reference sequence AAA52484.1.

As used herein, the term "activated protein C" or "APC" has its general meaning in the art and refers to any soluble polypeptide present in the blood sample that is derived from the human APC, which is characterized by the NCBI reference sequence NP_000303.

As used herein, the term "endothelial protein C receptor" or "EPCR" has its general meaning in the art and refers to any soluble polypeptide present in the blood sample that is derived from the human EPCR, which is characterized by the NCBI reference sequence NP_006395.

As used herein, the term "tissue factor pathway inhibitor" or "TFPI" has its general meaning in the art and refers to any soluble polypeptide present in the blood sample that is derived from the human TFPI, which is characterized by the NCBI reference sequence NP_001027452.

Standard methods of determining the level of a soluble marker typically involve contacting the blood sample obtained from the patient with a binding partner specific for said marker. In some embodiments, the binding partner may be an antibody that may be polyclonal or monoclonal, preferably monoclonal, directed against the specific soluble marker. Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies of the invention or a fragment thereof can be prepared and isolated using any technique that provides for the production of antibody molecules by continuous cell lines in culture. Techniques for production and isolation include but are not limited to the hybridoma technique; the human B-cell hybridoma technique; and the EBV-hybridoma technique. In some embodiments, the binding partner may be an aptamer. Aptamers are a class of molecule that represent an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library. In some embodiments, the binding partner of the invention is labelled with a detectable molecule or substance, such as a chromogenic substrate, a fluorescent molecule, a radioactive molecule or any other labels known in the art. Labels are known in the art that generally provide (either directly or indirectly) a signal. As used herein, the term "labelled", with regard to the antibody or aptamer, is intended to encompass direct labelling of the antibody or aptamer by coupling (i.e., physically linking) a detectable substance, such as a radioactive agent, an enzyme (e.g. horseradish peroxidase, or alkaline phosphatase) or a fluorophore (e.g. fluorescein isothiocyanate (FITC) or phycoerythrin (PE) or Indocyanine (Cy5) or allophycocyanin) to the antibody or aptamer, as well as indirect labelling of the probe or antibody by reactivity with a detectable substance. An antibody or aptamer of the invention may be labelled with a radioactive molecule by any method known in the art. For example radioactive molecules include but are not limited to radioactive atom for scintigraphic studies such as I¹²³, I¹²⁴, In¹¹¹, Re¹⁸⁶, Re¹⁸⁸. Preferably, the antibodies against the surface markers are already conjugated to a fluorophore (e.g. FITC-conjugated and/or PE-conjugated or allophycocyanin).

The aforementioned assays may involve the binding of the binding partners (i.e. antibodies or aptamers) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like. The solid surfaces are preferably beads. Since extracellular vesicles have a diameter of roughly 0.1 to 1 µm, the beads for use in the present invention should have a diameter larger than 1 µm. Beads may be made of different materials, including but not limited to glass, plastic, polystyrene, and acrylic. In addition, the beads are preferably fluorescently labelled. In some embodiments, an ELISA (enzyme-linked immunosorbent assay) method is used, wherein the wells of a microtiter plate are coated with a set of antibodies which recognize the said soluble marker of interest. The blood sample is then added to the coated wells. After a period of incubation sufficient to allow the formation of antibody-antigen complexes, the plate(s) can be washed to remove unbound moieties and a detectably labelled secondary binding molecule is added. The secondary binding molecule is allowed to react with any captured sample marker protein, the plate washed and the presence of the secondary binding molecule detected using methods well known in the art.

As used herein the term "extracellular vesicle" or "EV" has its general meaning in the art and denotes a plasma membrane vesicle shed from an apoptotic or activated cell. The size of extracellular vesicles ranges from 0.1 µm to 1 µm in diameter. The surface markers of extracellular vesicles are the same as the cells from which they originated. As used herein, the term "endothelial-derived extracellular vesicle" refers to an extracellular vesicles that derives from an endothelial cell. In particular endothelial-derived extracellular vesicles are characterized by the expression of tissue factor (TF) and annexin V. Thus in some embodiments, the method of the present invention comprises determining the level of endothelial TF+ and /or AnnV+ extracellular vesicles. Standard methods for determining the level of extracellular vesicles in a blood sample are well known in the art. For example the methods may consist in collecting a population of extracellular vesicles from a patient and using differential binding partners directed against the specific surface markers of said extracellular vesicles, wherein extracellular vesicles are bound by said binding partners to said surface markers. According to the invention, methods of flow cytometry are preferred methods for determining the level of extracellular vesicles in the blood sample obtained from the patient. For example, fluorescence activated cell sorting (FACS) may be therefore used to separate in the blood sample the desired extracellular vesicles. In another embodiment, magnetic beads may be used to isolate extracellular vesicles (MACS). For instance, beads labelled with monoclonal specific antibodies may be used for the positive selection of extracellular vesicles. Other methods can include the isolation of extracellular vesicles by depletion of non-extracellular vesicles components (negative selection). For example, extracellular vesicles may be excited with 488 nm wavelength light and logarithmic green and red fluorescences of FITC, PE and allophycocyanin may be measured through 530/30 nm, 585/42 nm and 660/20 nm bandpass filters, respectively. The absolute number of extracellular vesicles may then be calculated through specific software useful in practicing the methods of the present invention. Accordingly, in a specific embodiment, the method of the invention comprises the steps of obtaining a blood sample as above described; putting said prepared sample into a container; adding both labeled antibodies against surface markers that are specific to extracellular vesicles of interest, and a known concentration of fluorescent solid surfaces; performing a FACS analysis on the prepared sample in order to calculate the absolute number of extracellular vesicles therein.

As used herein, the term "DNA-histone complexes" has its general meaning in the art and refers to fibrillary networks of DNA, nuclear proteins including histones and granular proteins that are released from neutrophils during a complex cell death signaling pathway termed NETosis. Thus in some embodiments, the method of the present invention comprises determining the circulating level of DNA-histone complexes. Standard methods for determining the level of these complexes are ELISA (enzyme-linked immunosorbent assay) methods, wherein the wells of a microtiter plate are coated with a set of antibodies which recognize histones. The blood sample is then added to the coated wells. After a period of incubation sufficient to allow the formation of antibody-antigen complexes, the plate(s) can be washed to remove unbound moieties and a detectably labelled anti-DNA is added. The anti-DNA is allowed to react with any captured sample marker protein, the plate washed and the presence of anti-DNA detected using methods well known in the art.

Typically, the predetermined reference value is a threshold value or a cut-off value. Typically, a "threshold value" or "cut-off value" can be determined experimentally, empirically, or theoretically. A threshold value can be also arbitrarily selected based upon the existing experimental and/or clinical conditions, as would be recognized by an ordinary person skilled in the art. For example, retrospective measurement of thrombin generation capacities or levels of soluble markers in properly banked historical patient samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after quantifying the expression level in a group of reference, one can use algorithmic analysis for the statistic treatment of the determined levels in samples to be tested, and thus obtain a classification standard having significance for sample classification. The full name of a ROC curve is Receiver Operator Characteristic Curve, which is also known as receiver operation characteristic curve. It is used mainly for clinical biochemical diagnostic tests. A ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc. In some embodiments, high statistical significance values (e.g. low P values) are obtained for a range of successive arbitrary quantification values, and not only for a single arbitrary quantification value. Thus, in some embodiments, instead of using a definite predetermined reference value, a range of values is provided. Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P value) are retained, so that a range of quantification values is provided. This range of quantification values includes a "cut-off value as described above. For example, according to this specific embodiment of a "cut-off" value, the patient's state can be determined by comparing the capacity or level with the range of values which are identified. In some embodiments, a cut-off value thus consists of a range of quantification values, e.g. centered on the quantification value for which the highest statistical significance value is found (e.g. generally the minimum P value which is found). For example, on a hypothetical scale of 1 to 10, if the ideal cut-off value (the value with the highest statistical significance) is 5, a suitable (exemplary) range may be from 4-6. For example, a patient may be assessed by comparing values obtained by determining capacities or levels, where values greater than 5 reveal that the patient exhibits a hypercoagulable state and values less than 5 reveal that the patient does not exhibit features of a hypercoagulable state. In some embodiments, a patient may be assessed by comparing values obtained by measuring capacities or levels and comparing the values on a scale, where values above the range of 4-6 indicate that the patient exhibits a hypercoagulable state and values below the range of 4-6 indicate that the patient does not exhibit a hypercoagulable state, with values falling within the range of 4-6 indicate that further investigation is needed for concluding that the patient is in a hypercoagulable state.

The method of the present invention is particularly suitable for determining whether the patient is at risk of being rehospitalized. As used herein, the term "rehospitalized" denotes a patient who has been hospitalized for heart failure and who is again hospitalized. In this context the patient is hospitalized again for the same disease. In other words, the term "rehospitalized" denotes further hospitalization due to heart failure.

The method of the present invention is also particularly suitable for determining whether a patient is eligible for a treatment with an anticoagulant. As used herein, the term "anticoagulant" has its general meaning in the art and refers to a compound which is capable of preventing or inhibiting blood coagulation. Various compounds have been described as anticoagulants which affect one or more enzymes or auxiliary substances of the coagulation cascade. Coumarins, e.g., are plant-derived vitamin k antagonists which deplete the organism of the active form of vitamin K which is required as an auxiliary substance for thrombin and factors VII, IX and X activities. Typical coumarins include warfarin, acenocoumarol, phenprocoumon, atromentin, brodifacoum or phenindione. Heparins are highly sulfated glycosaminoglycanes and resemble another class of naturally occurring anticoagulants. They activate antithrombin which blocks the activity of thrombin and other enzymes of the coagulation cascade including factor Xa and, thereby, inhibit fibrin clot formation. Typically, low molecular weight heparin (LMWH) or unfractionated heparin (UFH) is used as heparin in anticoagulation therapy. Also heparanoids are used in anticoagulation therapy such as Danaparoid (also called Orgaran). In some embodiments, the anticoagulant is a factor Xa inhibitor. As used herein, the term "factor Xa inhibitor" refers to the ability of a compound to alter the function of factor Xa. A factor Xa inhibitor may block or reduce the activity of factor Xa by forming a reversible or irreversible covalent bond between the inhibitor and factor Xa or through formation of a noncovalently bound complex. In some embodiments, inhibition of factor Xa may be assessed using the method described in Wong et al, Journal of Thrombosis and Haemostasis 2008, 6(5), 820-829; Weitz et al, Thromb. Haemost. 2006, 96(3), 274-284; Turpie, A., Arterioscler Thromb Vasc Biol. 2007, 27, 1238-1247; Turpie, A., European Heart Journal 2007, 29, 155-165; and Jiang, et al., Thrombosis and Haemostasis 2009, 101(4), 780-782. Examples of factor Xa inhibitors include but are not limited to tamixaban, rivaroxaban, fondaparinux, and idraparinux.

The results obtained by the inventors also reveal that the patient who exhibits a hypercoagulable state can be eligible for an anticoagulant therapy which consists in administrating a therapeutically effective amount of a DNase. Any suitable DNase may be used in the present invention. The DNase will most preferably be a DNase I (EC 3.1.21.1). It may, however, in some embodiments be a DNase II (EC 3.1.21.1). DNases occur in a number of species and any DNase capable of cleaving DNA may be used in the invention. The DNase may be from an animal source such as of bovine or porcine origin. It may be of plant, fungal, or microbial origin. However, typically and most preferably the DNase is of human origin and is preferably a recombinant human DNase. Commercially available DNase preparations such as Dornase^{™} and Pulmozyme^{™} may be used in embodiments of the invention. In some embodiments, the DNase has DNA hydrolytic activity, for example in the case of DNase I it may hydrolyse DNA to give 5'-phosphate nucleotides and in the case of DNase II it may hydrolyse DNA to give 3' phosphate nucleotides. A fluorescence-based assay using, for example, Hoechst Stain may be used such as that which was detected in Labarce & Paiden, 1980, Anal. Biochem., 102:344-352 to assay for DNA hydrolysis. Hydrolytic activity may be assessed in a variety of ways known in the art including analytical polyacrylamide and agarose gel electrophoresis, hyperchromicity assay (Kunitz, J. Gen. Physiol. 33:349-362 (1950); Kunitz, J. Gen. Physiol. 33:363-377 (1950)) or methyl green assay (Kurnick, Arch. Biochem. 29:41-53 (1950); Sinicropi, et al., Anal. Biochem. 222:351-358 (1994)). The breakdown of DNA molecules from high to lower molecular weight forms may be monitored preferably by techniques such as agarose gel electrophoresis. Control experiments in the absence of the enzyme and/or in the presence of a protein known to possess DNase activity may be performed. DNases are known to be prone to deamidation. Asparagine residues and in particular the asparagine at amino acid positions 7 and 74 of the mature human DNase I are prone to deamidation. This process converts the asparagine residues in question to aspartic acid or iso-aspartate residues. Deamidation reduces the activity of the enzyme and this is particularly the case for deamidation at the asparagine at amino acid position 74 of mature human DNase I. Techniques are available for removing deamidated forms of the enzymes to leave the amidated forms and these may be employed to prepare DNases for use in the invention. These techniques may include tentacle cation exchange (TCX) or affinity purification using DNA to purify the amidated forms of the enzyme which are still capable of binding DNA. A DNase preparation for use in the invention may typically comprise from 85 to 100% (preferably from 90 to 100%), more preferably from 95 to 100% and even more preferably from 99 to 100%) amidated, or partially amidated, enzyme by weight. In particular these percentages refer to the amount of wholly amidated enzyme i.e. with all of the residues which are naturally amidated being amidated. They will typically have more than 95%, preferably more than 99% and even more preferably more than 99.9% of the DNase in an amidated form. In particular, these values refer to values at the time of production or to their values from one month to a year, preferably from two to six months and more preferably from three to four months after production. They may refer to the value during any point of the shelf-life of the product. Variants of naturally occurring or known DNases may be used in the invention. Thus the term DNase encompasses such variants. The term "variants" refers to polypeptides which have the same essential character or basic biological functionality as DNase. The essential character of a DNase is phosphodiesterase activity and the ability to hydrolyse DNA. Assays for measuring DNA cleavage are described herein and these may be used to determine whether a variant has hydrolytic activity. The sequence of the DNase may be modified so that it has extended half-life. For example the sequence of the enzyme may be changed to remove recognition sequences for certain proteases and in particular those derived from inflammatory cells. The DNase employed in the invention may also be chemically modified to alter its properties such as, for example, its biological half-life. To achieve this covalent modifications may be introduced by reacting targeted amino acid residues of the native or variant DNase with an organic derivatising agent that is capable of reacting with selected amino acid side-chains or N- or C-terminal residues. Suitable derivatising agents and methods are well known in the art. Residues which in particular may be derivatised include cysteinyl residues (most commonly by reaction with α-haloacetates), histidyl residues (by reaction with diethylpyrocarbonate at pH 5.5-7.0), lysinyl and amino terminal residues (by reaction with succinic or other carboxylic acid anhydrides), arginyl residues (by reaction with reagents such as phenylglyoxal, 2,3-butanedione, 1,2-cyclohexanedione, and ninhydrin). Carboxyl side groups (aspartyl or glutamyl) may be selectively modified by reaction with carbodiimides or may be converted to asparaginyl and glutaminyl residues by reaction with ammonium ions. The covalent attachment of agents such as polyethylene glycol (PEG) or human serum albumin to the DNases may reduce their immunogenicity and/or toxicity of the variant and/or prolong its half-life and hence may be used in the invention. In some embodiments of the invention the DNase may be directly conjugated to the glycosaminoglycan or joined through an intermediate molecule.

The results obtained by the inventors also reveal that the patient who exhibits a hypercoagulable state can be eligible for an anticoagulant therapy which consists in administrating a P-selectin antagonist. Said antagonist can indeed be suitable for preventing the deleterious effects mediated by the endothelial derived extracellular vesicles present in the blood stream of the patient. As used herein, the term "P-selectin" has its general meaning in the art and refers to a 140 kDa protein expressed by human platelets and endothelial cells, as described by Hsu-Lin et al, J Biol Chem 259: 9121 (1984), and Mc Ever et al, J Clin Invest 84:92 (1989). The term is also known as CD62P, GMP-140, PADGEM, and LECAM-3. As used herein, the term "P-selectin antagonist" includes any agent which is capable of antagonizing P-selectin, e.g., by inhibiting interaction between P-selectin and a P-selectin glycoprotein ligand-1, e.g., by inhibiting interactions of P-selectin expressing endothelial cells and activated platelets with PSGL-1 expressing leukocytes. In some embodiments, the P-selectin antagonist is an antibody against P-selectin. Antibodies against P-Selectin are known from, e.g., US patent 4,783,399, WO 93/06863, Geng et al (J. Biol. Chem., 266 (1991) 22313-22318), WO 93/21956, WO 2005/100402 and WO2008069999. In some embodiments, the anti-P-selectin of the present invention is SEG101 also named crizanlizumab.

As used herein, the term "treatment" or "treat" refer to both prophylactic or preventive treatment as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

The invention will be further illustrated by the following figures and examples. However, these examples and figures should not be interpreted in any way as limiting the scope of the present invention.

### FIGURES:

**Figure 1****. Acute decompensated heart failure increases plasma thrombin generation.** Representative thrombin generation curve in platelet-rich plasma. Thrombin generation was triggered by 0.5 pM tissue factor and monitored with the calibrated automated thrombogram assay.
**Figure 2****. Acute decompensated heart failure impairs activated protein C sensitivity.** Inhibition of endogenous thrombin potential (ETP) by activated protein C (APC) in platelet-rich plasma.
**Figure 3****. Levels of circulating markers of endothelial activation such as von Willebrand factor (VWF) are elevated patients with acute decompensated heart failure.**
**Figure 4****. The concentration of procoagulant endothelium-derived extracellular vesicles is elevated in patients with acute decompensated heart failure.** Extracellular vesicle-related procoagulant activities rely on phospholipid-dependent potential detected by annexin V binding.
**Figure 5****. Plasma levels of DNA-histone complexes are elevated in patients with acute decompensated heart failure.**

### EXAMPLES:

### EXAMPLE 1:

### Material & Methods

### Collection of blood samples

Venous blood samples were collected into Monovette^{®} (Sarstedt, Nümbrecht, Germany) syringes containing 1/10 volume of 0.10⁶ M sodium citrate. All analyses were performed within two hours after blood collection. Platelet-Rich Plasma (PRP) was prepared by blood centrifugation at 190g for 10 min at 20°C. The PRP was recovered and adjusted to 150 G/L by the addition of platelet-poor plasma obtained by centrifugation of the remaining blood at 1750g for 10 min at 20°C. Platelet-free Plasma (PFP) was obtained from platelet-poor plasma centrifuged at 13000g for 30 min at 4°C. PFP was immediately frozen at -80°C and thawed 15 min at 37°C when needed. For extracellular vesicle enumeration and characterization, blood samples were subjected to two successive centrifugations at 2500g for 15 min and the platelet-depleted plasma (PDP) was prepared and stored at -80°C until use.

### Thrombin generation assays

Calibrated automated thrombogram (CAT) was performed at 37°C in a microtiter plate fluorometer (Fluoroskan Ascent, ThermoLabsystems, Helsinki, Finland) using a dedicated software program (Thrombinoscope BV, Maastricht, The Netherlands).¹⁰ Coagulation was triggered with 0.5 pM recombinant human tissue factor (TF) (Innovin^{®}, Dade Behring). For measurements in PFP, a mixture of phosphatidyl-choline (PC)/serine (PS)/ethanolamine (PE) (PC/PS/PE, 60/20/20, mole %) at a final concentration of 4 µM was added. Thrombin generation curves were recorded in triplicate using PRP in the absence or in the presence of in-house activated protein C (APC) at several final concentrations (6.7, 13.9, 25 and 65 nM) and PFP in the absence or in the presence of in-house APC at several final concentrations (0.85, 1.7, 6.7 and 13.9 nM). The total amount of thrombin activity (i.e. the endogenous thrombin potential, ETP) was assessed as the area under the curve. The APC concentration that produced a 50% inhibition of ETP was defined as IC₅₀-APC.¹¹

### Hemostatic variables and endothelial markers

All measurements were performed in platelet-poor plasma. Soluble TM, soluble EPCR, free tissue factor pathway inhibitor (TFPI), von Willebrand factor (VWF) and factor VIII (FVIII) were measured by ELISA kits (Asserachrom, Diagnostica Stago^{™}, Asnières, France).

### Cell-derived extracellular vesicles

The fluorescent antibodies, CD41-phycoerythrin (CD41-PE), CD144-PE, CD14-PE and allophycocyanin-EPCR were purchased from BD Pharmingen^{™}. The other antibody against TF, clone TF9-10H10, allophycocyanin-TF was from Biotechne^{™}, and annexin-V (AnnV)-FITC from Fisher Scientific^{™}. MPs were enumerated by high-sensitivity flow cytometry using a standardized procedure:¹² plasma (20 µL) was incubated with the optimal concentration of specific antibody plus 10 µL of AnnV-FITC. Stained samples were analyzed on a GALLIOS instrument (Beckman-Coulter^{™}, Miami, FL, USA).

### Results

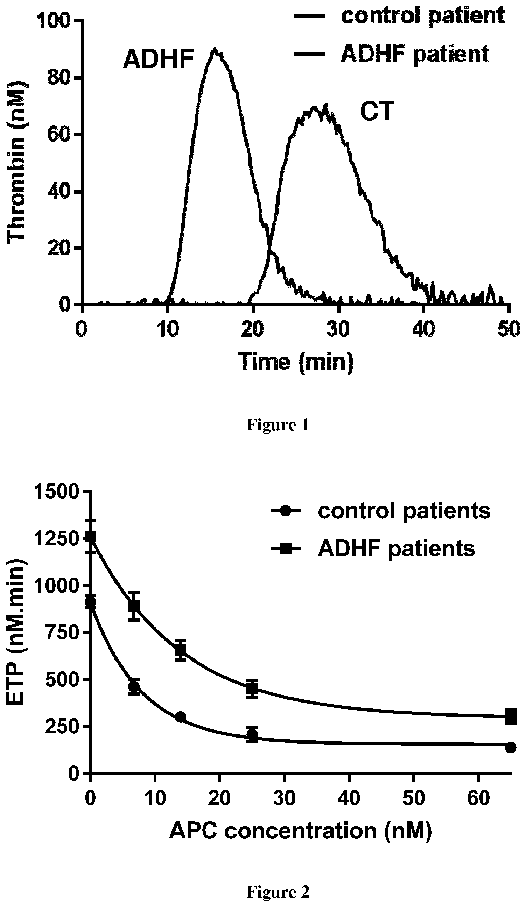

Compared to controls, endogenous thrombin potential (ETP) was higher in ADHF patients at hospital admission using platelet-rich and platelet-poor plasmas and remained increased during the hospitalization period (Figure 1).

The activated protein C (APC) concentration inhibiting thrombin generation in platelet-rich plasma by 50% was also higher at hospital admission (Figure 2).

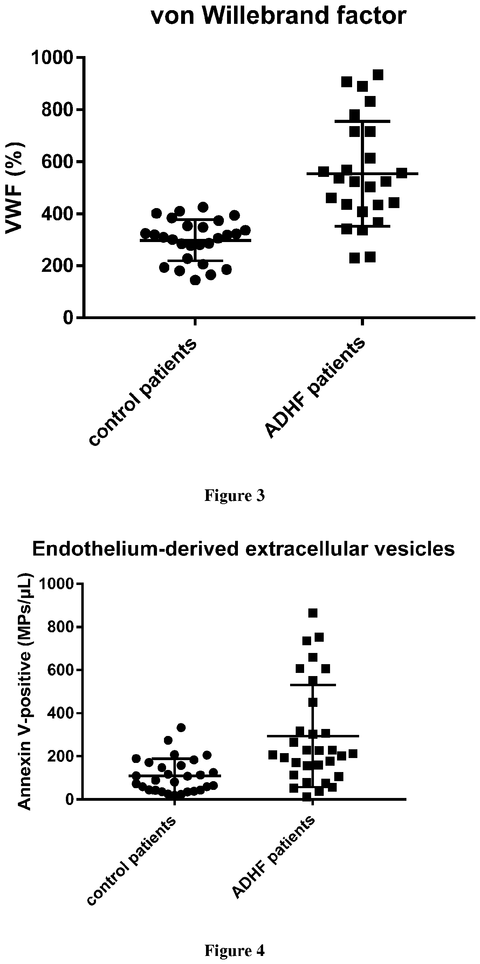

Soluble markers of endothelial dysfunction including von Willebrand factor (Figure 3), factor VIII, free tissue factor pathway inhibitor and the soluble forms of endothelial protein C receptor and thrombomodulin were higher in ADHF patients at hospital admission.

Higher concentrations of annexin-V/tissue factor (TF)-positive endothelium-derived extracellular vesicles were found in ADHF patients at hospital admission compared to controls (Figure 4).

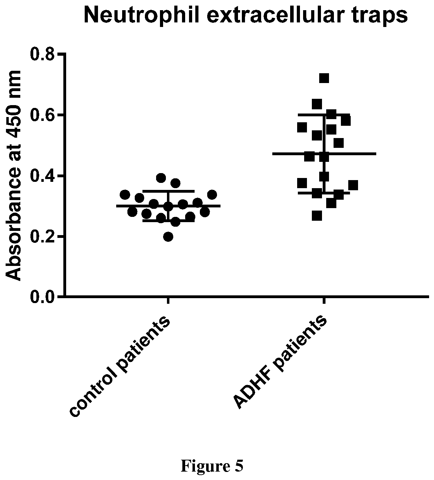

Plasma levels of DNA-histone complexes as an indirect measure of the highly complex neutrophil death signaling pathway (NETosis) are elevated in ADHF patients at hospital admission (Figure 5).

### EXAMPLE 2:

### Methods

### Patients and controls

The study was a single center prospective study of 34 consecutive patients admitted to a cardiology department for ADHF (all in New York Heart Association NYHA functional class III or IV) defined in accordance with the European Society of Cardiology guidelines as the rapid onset/progression of HF symptoms and signs secondary to abnormal cardiac function requiring hospital admission. Blood was collected at hospital admission, at the day of discharge and after hospital discharge following at least six weeks of clinical stability. Hemodynamic measurements were a routine part of clinical patient assessment and the HF therapy during hospital stay and at discharge was at the discretion of the treating physician. A group of 30 patients with stable chronic HF (CHF) recruited from outpatient clinics was also included to identify differences with the acute phase of decompensated HF. The study included 30 control in patients (CT) with preserved ventricular function referred to the department of cardiology for a scheduled medical assessment and who did not experience HF but had similar patterns of comorbidities, risk factors (eg, hypertension, diabetes and smoking) and background medication to clarify the potential confounding contribution of hospital stay. Exclusion criteria for all study groups were factors that could affect coagulation: infectious and inflammatory disorders, neoplasia, serum creatinine >200 µmοl/L, use of steroid drugs, acute coronary syndrome, atrial fibrillation and all comorbidities requiring therapeutic anticoagulation. Exclusion criteria during hospital stay were worsening HF requiring assist device or heart transplant. All ADHF patients received a venous thromboembolism (VTE) prophylaxis (enoxaparin 4000 IU once daily). Blood collection was performed before VTE prophylaxis at admission and 36 hours after the last enoxaparin injection at the day of discharge. No VTE prophylaxis was administered in CT and CHF patients. The study protocol was approved by the local ethics committee (Comité de Protection des Personnes agreement 15/10/2014). All study patients provided written informed consent.

### Collection of blood samples

Venous blood samples were collected into Monovette^{®} (Sarstedt, Nümbrecht, Germany) syringes containing 1/10 volume of 0.106 M sodium citrate. All analyses were performed within two hours after blood collection. Platelet-Rich Plasma (PRP) was prepared by blood centrifugation at 190g for 10 min at 20°C. The PRP was recovered and adjusted to 150 G/L for CAT by the addition of platelet-poor plasma obtained by centrifugation of the remaining blood at 1750g for 10 min at 20°C. Platelet-free Plasma (PFP) was obtained from platelet-poor plasma centrifuged at 13000g for 30 min at 4°C. PFP was immediately frozen at -80°C and thawed 15 min at 37°C when needed. For EV enumeration and characterization, blood was subjected to two successive centrifugations at 2500g for 15 min and the platelet-depleted plasma (PDP) was prepared and stored at -80°C until use.

### Thrombin generation assays

CAT was performed at 37°C using a dedicated software program (Thrombinoscope BV, Maastricht, The Netherlands) as reported previously. Coagulation was triggered with 0.5 pM recombinant human tissue factor (TF) (Innovin^{®}, Dade Behring). For measurements in PFP, a home-made mixture of phosphatidyl-choline/serine/ethanolamine (60/20/20, mole %) at a final concentration of 4 µM was added. Thrombin generation curves were recorded in triplicate in the absence or in the presence of in-house APC at several final concentration (6.7, 13.9, 25 and 65 nM for PRP and 0.85, 1.7, 6.7 and 13.9 nM for PFP). The total amount of thrombin activity (i.e. the endogenous thrombin potential, ETP) was assessed as the area under the curve. The APC concentration that produced a 50% inhibition of ETP was defined as IC50-APC.

### Quantification of hemostatic variables and endothelial markers

All measurements were performed in PDP. Soluble thrombomodulin (TM), soluble endothelial protein C receptor (EPCR), free tissue factor pathway inhibitor (TFPI), von Willebrand factor (VWF) and factor VIII (FVIII) were measured by ELISA kits (Asserachrom, Diagnostica Stago^{™}, Asnières, France).

### Measurement of endothelium-derived extracellular vesicles

The fluorescent antibodies, CD144-phycoerythrin (CD144-PE) and allophycocyanin EPCR were purchased from BD Pharmingen. The other antibody against TF, clone TF9-10H10, allophycocyanin-TF was from Biotechne^{™}, and annexin-V (AnnV)-FITC from Fisher Scientific^{™}. EVs were enumerated by high-sensitivity flow cytometry using a standardized procedure: plasma (20 µL) was incubated with the optimal concentration of specific antibody plus 10 µL of AnnV-FITC. Stained samples were analyzed on a GALLIOS instrument (Beckman-Coulter^{™}, Miami, FL, USA).

### Determination of nucleosome plasma levels

Circulating levels of nucleosomes were measured in PDP by a specific ELISA using a pair of antibodies directed against the histone and DNA components of nucleosomes (Cell Death Detection-ELISA plus, Roche Diagnostics, Sigma-Aldrich). Because the manufacturer did not provide a standard curve, a reference material containing high amounts of endogenous nucleosomes was used and nucleosome values were reported in arbitrary units.

### Statistical analysis

Categorical data are presented as numbers (%); continuous data are presented as means±standard error (SD). Differences in categorical values were performed using Pearson's 2 test or non-parametric Fisher's exact test as appropriate. Continuous variables were compared using the using Wilcoxon tests. The variation of the studied variables in ADHF patients throughout the follow-up period was assessed by using the Friedman test. Correlations were assessed by calculating the Spearman correlation coefficient. Alpha risk was fixed to 5% for all analysis. Analyses were done using SAS 9.4 (SAS Institute, Cary, NC, USA).

### Results

### Baseline clinical characteristics

The ADHF group at baseline was similar to the non-HF cardiology patients (CT) group with regard to the sex-ratio, comorbidities and risks factors (Table 1). ADHF had decreased left ventricular ejection fraction and increased left ventricular end diastolic volume and pulmonary arterial pressures. Serum B-type natriuretic peptide (BNP) and C-reactive protein (CRP) levels were higher in ADHF patients than in controls. No significant difference was found in the use of active cardiovascular medication except for diuretics, which were more often used in ADHF patients. Five ADHF patients died within three months after hospital discharge without available biological follow-up. Control CHF patients differed substantially from ADHF patients with respect to hypertension and diabetes, which were more frequent in ADHF patients. As expected, levels of BNP and CRP were higher in ADHF patients than in CHF patients. Patients with CHF and ADHF presented similar reduced kidney function, as measured by GFR (<60 mL/min), compared with controls. Leukocyte and platelet counts as well as fibrinogen levels were not different in all groups.

### In vitro thrombin generation is accelerated and increased in ADHF patients at hospital admission

At hospital admission, ETP in the presence of platelets (PRP adjusted at the same platelet count) was higher whereas lag-time and time to peak were markedly but not significantly shorter for ADHF patients compared to controls (Table 2 and data not shown). In absence of platelets (PFP), thrombin peak, ETP and velocity were increased and lag-time and time to peak were shortened for ADHF patients compared to controls (Table 2 and data not shown). Compared to controls, ETP was increased in CHF patients only in the presence of platelets. There was no significant difference in ETP between CHF and ADHF patients at admission in the presence of platelets whereas ETP was higher in the absence of platelets of ADHF patients. Increase in ETP and/or thrombin peak values was maintained on the day of discharge but was lost at the post-discharge time-point (Table 2 and data not shown).

### APC sensitivity is impaired in ADHF patients at hospital admission

ETP in the presence of APC was consistently higher at hospital admission compared to controls (data not shown). The APC concentration (IC50-APC) that gave half maximum inhibition of ETP without APC was significantly increased in the presence of platelets at hospital admission compared to controls (Table 2). The IC50-APC in the absence of platelets tended to increase in ADHF patients as compared to controls but did not reach statistical significance. No differences were found between CHF patients and controls or ADHF patients at admission in the presence of platelets whereas IC50-APC in the absence of platelets was increased in ADHF compared to CHF patients. When ETP (without APC) and IC50-APC are combined using their arithmetic product, a procoagulant phenotype was confirmed by a near threefold value at hospital admission compared to the controls (Table 2). This parameter tended to increase in PRP of CHF patients as compared to controls but did not reach statistical significance. Compared to CHF patients, ETP×IC50-APC values were increased in the absence of platelets of ADHF patients at admission. ETP values in the presence of APC were shifted downwards on the post-discharge time-point compared to admission (data not shown). At hospital discharge, and even more so at the post-discharge time-point, the arithmetic product ETP×IC50-APC was decreased.

### Soluble markers of endothelial activation are elevated at the acute decompensated phases of HF

ADHF patients had higher plasma levels of sEPCR, sTM, VWF, free TFPI and FVIII at hospital admission versus controls (Table 2 and data not shown). The increase in FVIII paralleled the increase in VWF as revealed by similar values of the FVIII/VWF ratio. Levels of these factors were significantly higher at admission of ADHF patients compared with plasma levels in the CHF group. Only FVIII and thus FVIII/VWF ratio was elevated in CHF patients compared to controls. Elevation in these factors was maintained on the day of discharge but was lost on the postdischarge time-point except for free TFPI. Compared to controls, the concentrations of AnnV+ eEVs, TF+ eEVs and AnnV+/TF+ eEVs was significantly higher in ADHF patients at admission (Table 2 and data not shown). The concentration of TF+ eEVs in CHF patients significantly increased compared to controls. No difference in the concentrations of EPCR+ eEVs and AnnV+/EPCR+ eEVs was denoted between ADHF patients and controls or CHF patients (Table 2). The concentration of AnnV+ eEVs and TF+ eEVs in ADHF patients significantly decreased over the hospital course and was reduced on the post-discharge time-point compared to admission.

### Circulating nucleosomes are elevated in ADHF patients at hospital admission

We measured the plasma levels of nucleosomes in a subset of ADHF patients including all those followed up after discharge. Circulating nucleosomes were increased in ADHF patients at admission compared with controls and CHF patients (data not shown). At the day of discharge, levels of nucleosomes remained elevated whereas a significant decline was evident on the post-discharge time-point. Nucleosome levels at hospital admission were significantly correlated with IC50-APC and ETP × IC50-APC in the presence of platelets (r=0.59; p=0.013 and r=0.63; p=0.006 respectively) and AnnV+ eEVs (r=0.76; p=0.0004).

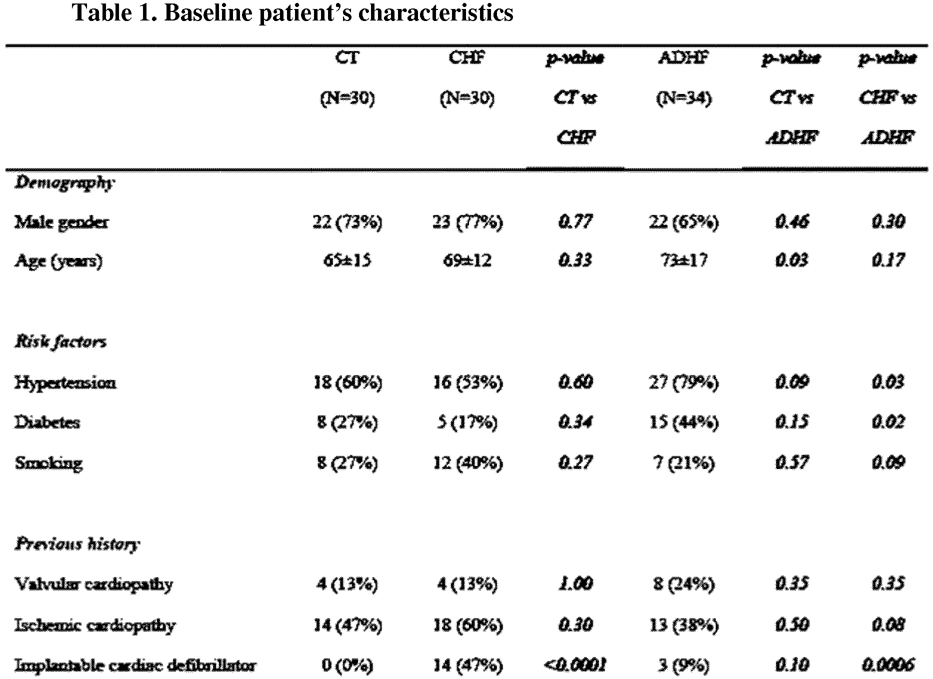

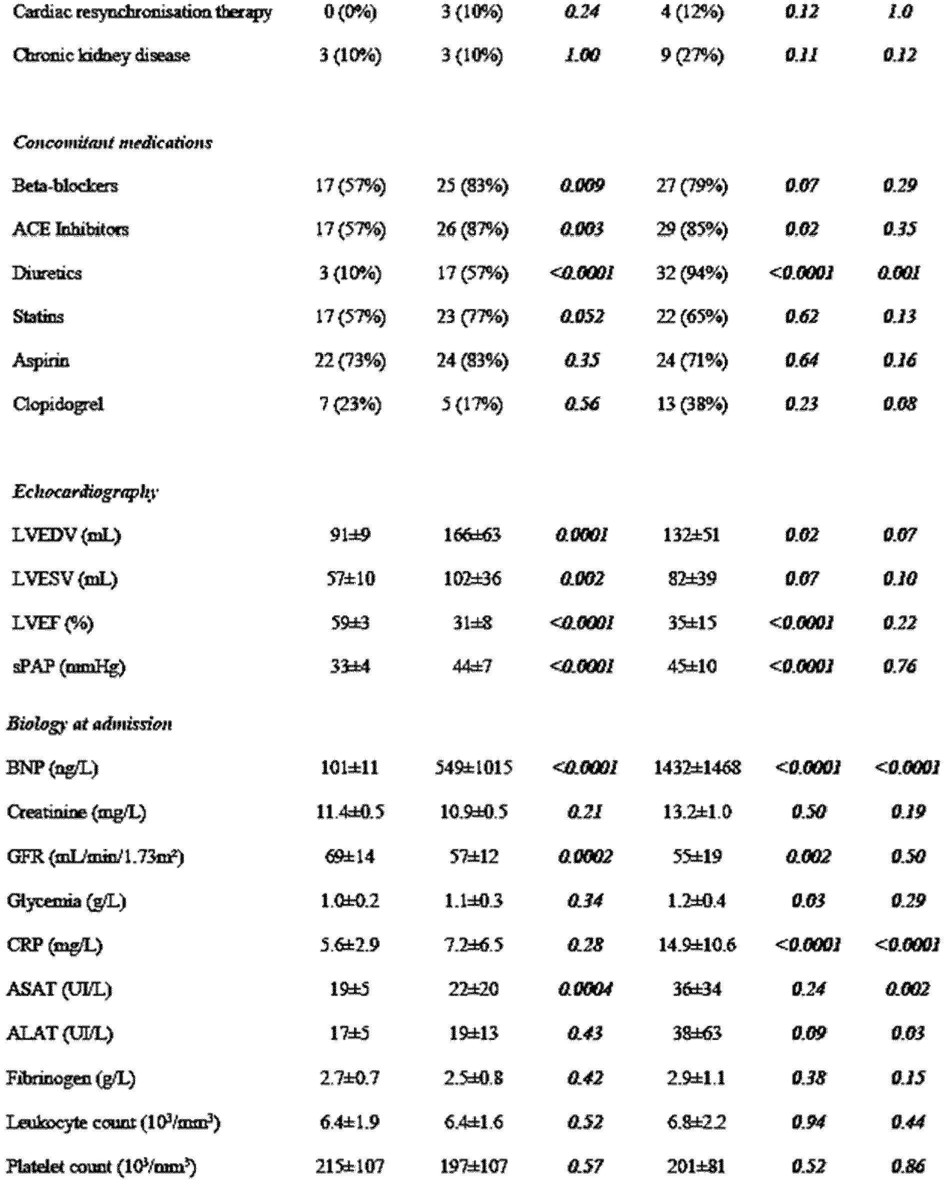

Results are mean±SD or frequency (percent). ACE indicates angiotensin-converting enzyme; LVEDV, left ventricular end diastolic volume; LVESV, left ventricular end systolic volume; LVEF, left ventricular ejection fraction; sPAP, pulmonary arterial pressures; BNP, B-type natriuretic peptide; GFR, glomerular filtration rate; CRP, C-reactive protein; ASAT, aspartate aminotransferase; ALAT, alanine aminotransferase.

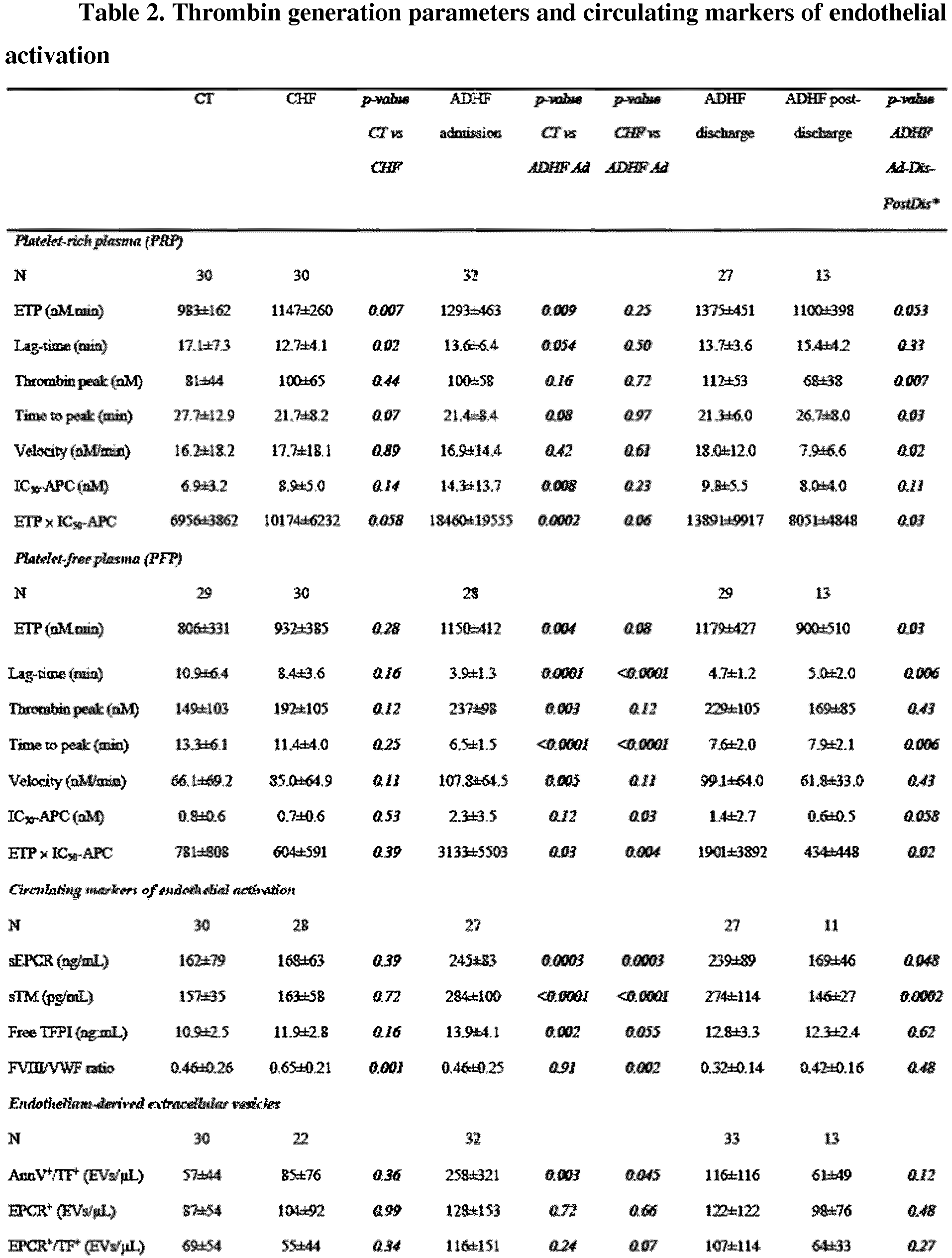

Values are means±SD. ETP indicates the endogenous thrombin potential in the absence of activated protein C (APC); IC₅₀-APC, the APC concentration needed to reduce ETP by 50%; EPCR, endothelial protein C receptor; TM, thrombomodulin; VWF, von Willebrand factor; FVIII, coagulation factor VIII; TFPI, tissue factor pathway inhibitor; EVs extracellular vesicles, AnnV, annexin V; TF, tissue factor. ^{∗} Friedman test for quantitative variables throughout the follow-up period.

### REFERENCES:

Throughout this application, various references describe the state of the art to which this invention pertains.
1. Kociol RD, Hammill BG, Fonarow GC, Klaskala W, Mills RM, Hernandez AF, Curtis LH. Generalizability and longitudinal outcomes of a national heart failure clinical registry: Comparison of Acute Decompensated Heart Failure National Registry (ADHERE) and non-ADHERE Medicare beneficiaries. Am Heart J. 2010; 160: 885-92. S0002-8703(10)00661-7 [pii] 10.1016/j.ahj.2010.07.020.
2. Giamouzis G, Kalogeropoulos A, Georgiopoulou V, Laskar S, Smith AL, Dunbar S, Triposkiadis F, Butler J. Hospitalization epidemic in patients with heart failure: risk factors, risk prediction, knowledge gaps, and future directions. J Card Fail. 2011; 17: 54-75. S1071-9164(10)01089-4 [pii] 10.1016/j.cardfail.2010.08.010.
3. Gurbel PA, Tantry US. Antiplatelet and anticoagulant agents in heart failure: current status and future perspectives. JACC Heart Fail. 2014; 2: 1-14. S2213-1779(13)00410-1 [pii] 10.1016/j.jchf.2013.07.007.
4. Gheorghiade M, Vaduganathan M, Fonarow GC, Greene SJ, Greenberg BH, Liu PP, Massie BM, Mehra MR, Metra M, Zannad F, Cleland JG, van Veldhuisen DJ, Shah AN, Butler J. Anticoagulation in heart failure: current status and future direction. Heart Fail Rev. 2013; 18: 797-813. 10.1007/s10741-012-9343-x.
5. Zannad F, Stough WG, Regnault V, Gheorghiade M, Deliargyris E, Gibson CM, Agewall S, Berkowitz SD, Burton P, Calvo G, Goldstein S, Verheugt FW, Koglin J, O'Connor CM. Is thrombosis a contributor to heart failure pathophysiology? Possible mechanisms, therapeutic opportunities, and clinical investigation challenges. Int J Cardiol. 2013; 167: 1772-82. S0167-5273(12)01647-6 [pii] 10.1016/j.ijcard.2012.12.018.
6. Zannad F, Greenberg B, Cleland JG, Gheorghiade M, van Veldhuisen DJ, Mehra MR, Anker SD, Byra WM, Fu M, Mills RM. Rationale and design of a randomized, double-blind, event-driven, multicentre study comparing the efficacy and safety of oral rivaroxaban with placebo for reducing the risk of death, myocardial infarction or stroke in subjects with heart failure and significant coronary artery disease following an exacerbation of heart failure: the COMMANDER HF trial. Eur J Heart Fail. 2015; 17: 735-42. 10.1002/ejhf.266.
7. Schoner A, Tyrrell C, Wu M, Gelow JM, Hayes AA, Lindner JR, Thornburg KL, Hasan W. Endocardial Endothelial Dysfunction Progressively Disrupts Initially Anti then Pro-Thrombotic Pathways in Heart Failure Mice. PLoS One. 2015; 10: e0142940. 10.1371/journal.pone.0142940PONE-D-15-35329 [pii].
8. Nozaki T, Sugiyama S, Sugamura K, Ohba K, Matsuzawa Y, Konishi M, Matsubara J, Akiyama E, Sumida H, Matsui K, Jinnouchi H, Ogawa H. Prognostic value of endothelial microparticles in patients with heart failure. Eur J Heart Fail. 2010; 12: 1223-8. hfq145 [pii] 10.1093/eurjhf/hfq145.
9. Martinod K, Wagner DD. Thrombosis: tangled up in NETs. Blood, 2014; 123: 2768-76.
10. Hemker HC, Giesen P, Al Dieri R, Regnault V, de Smedt E, Wagenvoord R, Lecompte T, Beguin S. Calibrated automated thrombin generation measurement in clotting plasma. Pathophysiol Haemost Thromb. 2003; 33: 4-15.
11. Regnault V, Beguin S, Wahl D, de Maistre E, Coenraad Hemker H, Lecompte T. Thrombinography shows acquired resistance to activated protein C in patients with lupus anticoagulants. Thromb Haemost. 2003; 89: 208-12.
12. Lacroix R, Robert S, Poncelet P, Kasthuri RS, Key NS, Dignat-George F. Standardization of platelet-derived microparticle enumeration by flow cytometry with calibrated beads: results of the International Society on Thrombosis and Haemostasis SSC Collaborative workshop. J Thromb Haemost. 2010; 8: 2571-4.

## Claims

1. A method for identifying whether a patient with acute decompensated heart failure (ADHF) exhibits a hypercoagulable state comprising i) determining thrombin generation capacity from a blood sample that has been obtained from the patient, ii) determining the level of at least one soluble biomarker of endothelial dysfunction, iii) comparing the capacity determined at step i) and the level determined at step ii) with their respective predetermined reference values and iv) concluding that the patient exhibits a hypercoagulable state when both the thrombin generation capacity and the level of soluble marker of endothelial dysfunction are higher than their respective predetermined reference values wherein:
- the thrombin generation capacity is determined by a calibrated automated thrombogram (CAT) assay and
- the at least one soluble biomarker of endothelial dysfunction is selected from the group consisting of von Willebrand factor, factor VIII, endothelial protein C receptor, tissue factor pathway inhibitor, endothelium-derived extracellular vesicles and circulating DNA-histone complexes.

2. The method of claim 1 wherein the blood sample is a platelet-rich plasma (PRP) sample or a platelet-poor plasma sample.

3. The method of claim 1 which comprises determining the Endogenous Thrombin Potential (ETP) in the blood sample obtained from the patient and comparing said value with a predetermined reference value.

4. The method of claim 1 wherein activated protein C (APC) is added to the blood sample and the APC concentration inhibiting thrombin generation by 50% is thus determined and compared to a predetermined reference value.

5. The method of claim 1 which comprises determining the level of a soluble marker by contacting the blood sample obtained from the patient with a binding partner specific for said marker.

6. The method of claim 1 which comprises determining the level of endothelial TF+ and/or AnnV+ extracellular vesicles.

## Patentansprüche

1. Verfahren zum Identifizieren, ob ein Patient mit akuter dekompensierter Herzschwäche (ADHF) einen hyperkoagulierbaren Zustand vorweist, umfassend i) Bestimmen von Thrombinerzeugungskapazität aus einer Blutprobe, die von dem Patienten erhalten wurde, ii) Bestimmen des Grads von mindestens einem löslichen Biomarker von endothelialer Dysfunktion, iii) Vergleichen der bei Schritt i) bestimmten Kapazität und dem bei Schritt ii) bestimmten Grad mit deren jeweiligen vorgegebenen Referenzwerten und iv) Schlussfolgern, dass der Patient einen hyperkoagulierbaren Zustand vorweist, wenn sowohl die Thrombinerzeugungskapazität als auch der Grad von löslichem Marker von endothelialer Dysfunktion höher als deren jeweilige vorgegebene Referenzwerte sind, wobei:
- die Thrombinerzeugungskapazität durch ein kalibriertes automatisiertes Thrombogramm- (CAT) -assay bestimmt wird, und
- der mindestens eine lösliche Biomarker von endothelialer Dysfunktion aus der Gruppe ausgewählt ist, die aus von-Willebrand-Faktor, Faktor VIII, enthothelialem Protein-C-Rezeptor, Gewebefaktorleitungsbahnhemmer, Endothel-abgeleiteten extrazellulären Vesikeln und zirkulierenden DNA-Histonkomplexen besteht.

2. Verfahren nach Anspruch 1, wobei die Blutprobe eine plättchenreiche Plasma-(PRP) -probe oder eine plättchenarme Plasmaprobe ist.

3. Verfahren nach Anspruch 1, das Bestimmen des endogenen Thrombinpotenzials (ETP) in der Blutprobe, die von dem Patienten erhalten wurde, und Vergleichen des Werts mit einem vorgegebenen Referenzwert umfasst.

4. Verfahren nach Anspruch 1, wobei aktiviertes Protein-C (APC) zu der Blutprobe hinzugefügt wird und die APC-Konzentration, die Thrombinerzeugung um 50% hemmt, folglich bestimmt und mit einem vorgegebenen Referenzwert verglichen wird.

5. Verfahren nach Anspruch 1, das Bestimmen des Grads eines löslichen Markers umfasst, indem die Blutprobe, die von dem Patienten erhalten wurde, mit einem Bindungspartner kontaktiert wird, der für den Marker spezifisch ist.

6. Verfahren nach Anspruch 1, das Bestimmen des Grads von endothelialen TF+ und/oder AnnV+ extrazellulären Vesikeln umfasst.

## Revendications

1. Procédé pour identifier si un patient souffrant d'une insuffisance cardiaque décompensée aiguë (ICDA) présente un état d'hypercoagulabilité comprenant i) la détermination de la capacité de génération de thrombine à partir d'un échantillon de sang prélevé sur le patient, ii) la détermination du taux d'au moins un biomarqueur soluble de dysfonctionnement endothélial, iii) la comparaison de la capacité déterminée à l'étape i) et du taux déterminé à l'étape ii) à leurs valeurs de référence prédéterminées respectives et iv) la conclusion que le patient présente un état d'hypercoagulabilité quand à la fois la capacité de génération de thrombine et le taux de marqueur soluble de dysfonctionnement endothélial sont supérieurs à leurs valeurs de référence prédéterminées respectives dans lequel :
- la capacité de génération de thrombine est déterminée par un test de thrombogramme automatisé étalonné (CAT) et
- le au moins un biomarqueur soluble de dysfonctionnement endothélial est sélectionné dans le groupe consistant en le facteur von Willebrand, le facteur VIII, le récepteur endothélial de la protéine C, l'inhibiteur de la voie du facteur tissulaire, les vésicules extracellulaires dérivées de l'endothélium et les complexes ADN-histone circulant.

2. Procédé selon la revendication 1, dans lequel l'échantillon de sang est un échantillon de plasma riche en plaquettes (PRP) ou un échantillon de plasma pauvre en plaquettes.

3. Procédé selon la revendication 1, qui comprend la détermination du potentiel endogène de thrombine (ETP) dans l'échantillon de sang prélevé sur le patient et la comparaison de ladite valeur à une valeur de référence prédéterminée.

4. Procédé selon la revendication 1, dans lequel une protéine C activée (PCA) est ajoutée à l'échantillon de sang et la concentration en PCA inhibant la génération de thrombine de 50% est ainsi déterminée et comparée à une valeur de référence prédéterminée.

5. Procédé selon la revendication 1, qui comprend la détermination du taux d'un marqueur soluble en mettant en contact l'échantillon de sang prélevé sur le patient avec un partenaire de liaison spécifique pour ledit marqueur.

6. Procédé selon la revendication 1, qui comprend la détermination du taux des vésicules endothéliales TF+ et/ou AnnV+ extracellulaires.
